# EUROPÄISCHE PATENTSCHRIFT

(11) **EP 3 620 208 B1**
(45) Veröffentlichungstag und Bekanntmachung des Hinweises auf die Patenterteilung: **06.09.2023**
(21) Anmeldenummer: 19183959.6
(22) Anmeldetag: 02.07.2019
(51) Int. Cl.: A61N 1/36, A61N 1/05, A61B 17/34, A61B 5/00, A61B 8/08, A61B 17/00, A61B 18/00

(54) **KANÜLE**
CANNULA
CANULE

(30) Priorität: 06.09.2018 DE 102018121733
(43) Veröffentlichungstag der Anmeldung: 11.03.2020
(73) Patentinhaber: Pajunk GmbH Medizintechnologie, 78187 Geisingen (DE)
(72) Erfinder: Hauger, Martin, 78166 Donaueschingen (DE); Pajunk-Schelling, Simone, 78187 Geisingen (DE)
(74) Vertreter: Westphal, Mussgnug & Partner Patentanwälte mbB

(56) Entgegenhaltungen:
- EP-A2- 1 611 922
- DE-A1-102016 110 379
- US-A1- 2008 200 835
- US-A1- 2010 204 568
- US-A1- 2014 025 039

## Beschreibung

Die vorliegende Erfindung betrifft eine Kanüle mit den Merkmalen des Patentanspruchs 1.

Aus dem Stand der Technik sind Kanülen in unterschiedlichen Ausgestaltungen vorbekannt. Kanülen umfassen typischerweise ein aus einem elektrisch leitfähigen Werkstoff, vorzugsweise Metall, hergestelltes Kanülenrohr, an dessen proximalen Ende ein Ansatz und an dessen distalen Ende eine Kanülenspitze angeordnet sind. Durch den Ansatz kann die Kanülenspitze durch ein medizinisches Personal zu Behandlungszwecken beispielsweise transkutan zu einem Nerv eines Patienten geführt werden, wobei die Lage der Kanülenspitze unter anderem durch elektrische Stimulationsreize festgestellt werden kann. Die elektrischen Stimulationsimpulse werden über ein Stimulationskabel zugeführt, welches das elektrisch leitende Kanülenrohr im Bereich eines proximalen Ansatzes der Kanüle kontaktiert. Solche Kanülen sind beispielsweise aus der US 7,022,115 B1, der EP 1 002 500 A1 und der DE 10 2016 110 379 A1 bekannt und werden insbesondere in der Anästhesie für die periphere Nervenblockade verwendet. Die DE 10 2016 110 379 A1 offenbart die Merkmale des Oberbegriffs des Patentanspruchs 1.

Um die genaue Position der Kanülenspitze bzw. des Kanülenrohrs in dem zu behandelnden Patienten bei der Therapie bzw. bei der Anästhesie zu beobachten und zu überprüfen, kommt vermehrt Ultraschall zum Einsatz. Dabei wird ein Ultraschallkopf auf die Körperoberfläche des Patienten aufgesetzt der Ultraschallsignale aussendet und die von dem metallischen Kanülenrohr reflektierten Ultraschallsignale empfängt. Die verbesserte Ultraschall-Sichtbarkeit der Kanüle, wie sie beispielsweise in der WO 2010/012 024 A1 beschrieben ist, hat zur Folge, dass die Anästhesisten sich zunehmend auf die Ultraschall-Ortung des Kanülenrohrs beschränken und auf die Elektrostimulation verzichten.

Weiteren Stand der Technik bilden die Druckschriften US 2014 015 039 A1 und US 2008 200 835 A1.

Es hat sich als nachteilig erwiesen, dass das medizinische Fachpersonal einerseits den Ultraschallkopf zum Prüfen und Beobachten der Position des Kanülenrohrs halten muss und darüber hinaus durch geeignete Bedienelemente einerseits die Zufuhr als auch die Aspiration einer Flüssigkeit durch die Kanüle, insbesondere eines Lokalanästhetikums, steuern und andererseits die elektrischen Stimulationsreize setzen muss. Bei den aus dem Stand der Technik bekannten Kanülen hat sich die Betätigung als umständlich erwiesen und erforderte erhöhten personellen Bedarf an medizinischem Personal bei der Behandlung des Patienten.

Hier setzt die vorliegende Erfindung an.

Der Erfindung liegen die Aufgaben zugrunde, eine verbesserte Kanüle zur Verfügung zu stellen, die zweckmäßigerweise die Nachteile der aus dem Stand der Technik bekannten Kanülen beseitigt und in zweckmäßiger Weise eine vereinfachte Bedienung durch das medizinische Personal sowie Kostenvorteile bei der Herstellung und Wiederverwendung einzelner Bestandteile vereint. Insbesondere soll die Handhabung der verbesserten Kanüle und des Ultraschallkopfes zum Beobachten und Prüfen der Position des Kanülenrohrs durch eine einzige Person erfolgen können, wobei sämtliche Funktionen der Kanüle, also das Steuern der Flüssigkeitszufuhr und/oder der Flüssigkeitsabführung bzw. des Zuspritzens und/oder des Aspirierens und das Setzen der Stimulationsimpulse sowie die Führung der Kanüle bzw. die Positionierung des distalen Endes des Kanülenrohrs an dem Nerv in einem einzigen Handgriff möglich sind. Durch die Kombination von Stimulation und Ultraschall soll mehr Sicherheit für Patienten und das medizinische Personal ermöglicht werden. Die bildliche sonographische Darstellung der individuellen Anatomie des Patienten und die zeitgleiche Kontrolle des Abstands der Kanüle zum Nerven mittels Stimulation ermöglichen nicht nur eine Optimierung der Punktionsgenauigkeit und somit beim Zuspritzen des Lokalanästhetikums eine optimale Wirkung und mengenmäßig optimalen Einsatz des Lokalanästhetikums, sondern bringen als Technik nachweislich auch einen zeitlichen Vorteil bei der Behandlung.

Diese Aufgaben werden erfindungsgemäß durch eine Kanüle mit den Merkmalen des Anspruchs 1 gelöst.

Die Unteransprüche haben weitere vorteilhafte Ausgestaltungen der Erfindung zum Inhalt.

Der wesentliche Gedanke der Erfindung beruht darauf, eine Kanüle nach Art eines modularen Systems mehrteilig auszubilden, wodurch diese je nach Anwendungszweck in einer ersten Aufbaustufe mit einem Steuerclip oder in einer zweiten Aufbaustufe zusätzlich mit einem Kontaktclip zur Elektrostimulation ausgestattet werden kann.

Die Kanüle mit den Merkmalen des Anspruchs 1 umfasst einen Kanülenkörper mit einem Kanülenrohr und einem an einem proximalen Ende des Kanülenrohrs angebrachten Ansatz und einen Steuerclip, aufweisend einen Befestigungskörper mit wenigstens einem elektrischen Bedienelement der zur Bildung einer ersten Aufbaustufe an dem Ansatz seitlich anbringbar ist.

Nach Maßgabe der vorliegenden Erfindung ist die erfindungsgemäße Kanüle in einer zweiten Aufbaustufe mit einem Kontaktclip versehen, der seitlich auf den Ansatz ergonomisch angeordnet wird. Der Kontaktclip umfasst ein Befestigungsteil mit wenigstens einem elektrischen Kontakt, wobei der Kontaktclip seitlich an den Ansatz zur Bildung der zweiten Aufbaustufe anbringbar ist und der elektrische Kontakt in der zweiten Aufbaustufe eine elektrische Verbindung zwischen einem Stimulationskabel und einer elektrisch leitenden Ader des Kanülenrohrs im Bereich des Ansatzes herstellt. In der zweiten Aufbaustufe kann die erfindungsgemäße Kanüle nach Art einer herkömmlichen aus dem Stand der Technik bekannten Kanüle zur Elektrostimulation verwendet werden, wobei bevorzugt in der zweiten Ausbaustufe das wenigstens eine Bedienelement des Kontaktclips zum Kontrollieren der elektrischen Stimulationsimpulse verwendet werden kann. Nach der Verwendung der erfindungsgemäßen Kanüle kann der Kontaktclip von dem Ansatz abgenommen werden und nach einer entsprechenden Reinigung für die Behandlung von weiteren Patienten verwendet werden.

Gemäß einer weiteren vorteilhaften Ausgestaltung der vorliegenden Erfindung weist das Befestigungsteil des Kontaktclips wenigstens ein Paar von federnden Rastarmen auf, die an dem Ansatz einrasten. Die Befestigung des Kontaktclips erfolgt folglich mittels einer Rastverbindung, wobei der Kontaktclip seitlich auf den Ansatz aufgesteckt wird und die federnden Rastarme jeweils mindestens eine Rastnase aufweisen, die in eine dazu korrespondierende Rastaussparung in den Ansatz eingreift und/oder die eine korrespondierende Kante des Ansatzes hintergreift. Die Rastarme legen sich beim Überführen des Kontaktclips an den Ansatz zur Bildung der zweiten Aufbaustufe diametral zueinander beiderseits an den Ansatz an, wobei die Aussparung für das Eingreifen des elektrischen Kontakts des Kontaktclips sich in dem Bereich des Ansatzes zwischen den Rastarmen befindet. Alternativ kann der Kontaktclip bzw. das Befestigungsteil des Kontaktclips eingerichtet sein, schwenkbar an dem Ansatz eingehängt und durch die federnden Rastarme an dem Ansatz arretiert zu werden.

Eine vorteilhafte Weiterbildung der vorliegenden Erfindung sieht vor, dass in der zweiten Aufbaustufe der elektrische Kontakt in eine Aussparung des Ansatzes eingreift und das Kanülenrohr elektrisch kontaktiert. Wird also der Kontaktclip mit dem Befestigungsteil an den Ansatz zur Bildung der zweiten Aufbaustufe angebracht, so greift der elektrische Kontakt in die Aussparung des Ansatzteils. Die Aussparung des Ansatzteils lässt das metallische Kanülenrohr bzw. die elektrisch leitende Ader des nicht-elektrisch leitfähigen Kanülenrohrs frei und ermöglicht eine elektrische Verbindung zwischen dem elektrischen Kontakt und dem metallischen Kanülenrohr bzw. der elektrisch leitfähigen Ader des Kanülenrohrs. Dementsprechend ist der elektrische Kontakt in dem Befestigungsteil des Kontaktclips auf der dem Ansatz zugewandten Seite angeordnet, wodurch die äußere Gestaltung des Ansatzes nicht beeinflusst wird und die Handhabung der erfindungsgemäßen Kanüle unbehindert ist und vollständig einer herkömmlichen und aus dem Stand der Technik bekannten Kanüle entspricht.

Es hat sich als vorteilhaft erwiesen, wenn der elektrische Kontakt zum elektrischen Kontaktieren des Kanülenrohrs als ein Schneidklemmkontakt ausgebildet ist, der beim Überführen des Kontaktclips in die zweite Aufbaustufe auf das Kanülenrohr gedrückt wird. Durch den Schneidklemmkontakt wird ein zuverlässiger elektrischer Kontakt mit dem Kanülenrohr bewirkt und sichergestellt, dass sich keine isolierende Schicht auf dem Kanülenrohr nachteilig auf die Übertragung der elektrischen Stimulationsimpulse von dem elektrischen Kontakt auf das Kanülenrohr bzw. die elektrisch leitende Ader des Kanülenrohrs auswirkt.

Darüber hinaus ist nach Maßgabe einer weiteren vorteilhaften Ausgestaltung der vorliegenden Erfindung der elektrische Kontakt mit einer Federzunge versehen, die eingerichtet ist, in die Aussparung des Ansatzes zu ragen und sich auf das Kanülenrohr zur Bildung einer elektrischen Verbindung anzulegen. Insbesondere hat es sich als vorteilhaft erwiesen, wenn das freie Ende der Federzunge scharfkantig ausgebildet ist, wodurch bei dem Anlegen der Federzunge auf das Kanülenrohr eine sichere elektrische Verbindung bewirkt wird.

Eine weitere vorteilhafte Ausgestaltung der vorliegenden Erfindung sieht vor, dass der Kontaktclip einen Steckverbinder aufweist, der eine elektrisch lösliche Verbindung mit dem Stimulationskabel herstellen kann. Insbesondere hat es sich dabei als vorteilhaft erwiesen, wenn der elektrische Kontakt eine weitere Federzunge an einem zweiten Ende aufweist, welche einen Klemmkontakt zur Bildung des elektrischen Steckverbinders bereitstellt. Dadurch ist ein besonders kompakt ausgebildeter Steckverbinder bereitgestellt, welcher den Anforderungen an eine platzsparende Ausgestaltung gerecht wird. Besonders bevorzugt ist dabei der Steckverbinder mittig zwischen den paarweise angeordneten Rastarmen angeordnet und wird weiter bevorzugt in der ersten und/oder der zweiten Aufbaustufe durch an dem Ansatz angeformte oder angearbeitete Haltestege formschlüssig gehalten.

Nach Maßgabe einer weiteren vorteilhaften Ausgestaltung der vorliegenden Erfindung ist das wenigstens eine Bedienelement ein elektrisches Bedienelement. Das Bedienelement ist weiter bevorzugt mit einer Steuerleitung verbunden, wobei die Steuerleitung mit einer Vorrichtung gekoppelt sein kann, welche die elektrischen Stimulationsimpulse erzeugt, die mittels des Stimulationskabels und des Kontaktclips auf die elektrisch leitende Ader des Kanülenrohrs übertragen werden. Darüber hinaus kann die Steuerleitung weiterhin bevorzugt mit der Vorrichtung oder einer weiteren Vorrichtung elektrisch verbunden sein, welche eingerichtet ist, eine Flüssigkeit zuzuführen oder aufzusaugen, welche durch das Kanülenrohr zugespritzt bzw. aspiriert werden soll. Dementsprechend ist das wenigstens eine Bedienelement des Steuerclips eingerichtet, einen in dem Stimulationskabel fließenden elektrischen Strom, der die Stimulationsimpulse darstellt, zu kontrollieren, insbesondere das Ein- und Ausschalten. Darüber hinaus kann das wenigstens eine Bedienelement dazu ausgebildet sein, die Stimulationsimpulse zu kontrollieren, insbesondere die Amplitude und die Frequenz.

Vorteilhaft ist es, wenn der Steuerclip wenigstens ein zweites elektrisches Bedienelement aufweist, durch welches beispielsweise die durch das Kanülenrohr geleitete Flüssigkeit, welche dem Patienten zugespritzt wird, kontrolliert werden kann. Insbesondere ist es vorteilhaft, wenn durch das wenigstens eine zweite elektrische Bedienelement das Zuspritzen und/oder das Aspirieren der Flüssigkeit ein- bzw. ausgeschaltet werden kann. Es kann darüber hinaus vorteilhaft sein, wenn das wenigstens eine zweite Bedienelement eingerichtet ist, die Zuspritz- oder Aspirationsrate, also die Menge an zugespritzter oder abgesaugter Flüssigkeit pro Zeiteinheit, zu beeinflussen. Dabei ist es weiterhin vorteilhaft, wenn die wenigstens zwei Rastarme des Befestigungskörpers des Steuerclips zu Befestigungszwecken an der Seite des Ansatzes den Ansatz und/oder den Kontaktclip umgreifen und bevorzugt eine Rastverbindung mit dem Kontaktclip und/oder dem Ansatz der Kanüle herstellen. Dabei ist insbesondere vorteilhaft, wenn die wenigstens zwei Rastarme des Befestigungskörpers des Steuerclips diametral zueinander beabstandet angeordnet sind und den Steuerclip und/oder den Ansatz umgreifen. Weiter bevorzugt befindet sich der Kontaktclip in der ersten Aufbaustufe bereits an dem Ansatz oder in dem Bereich des Steuerclips zwischen den wenigstens zwei Rastarmen. Diese konstruktive Maßnahme zur Befestigung des Steuerclips beeinflusst die äußere Gestaltung des Ansatzes bzw. der Kanüle nur geringfügig, wodurch die Handhabung der Kanüle unbehindert und insbesondere einhändig in einer Handgriffstellung erfolgen kann. Insbesondere ist dabei bevorzugt, wenn die wenigstens zwei federnden Rastarme des Steuerclips das Befestigungsteil des Kontaktclips umgreifen und die Steckrichtung des Kontaktclips und des Steuerclips die gleiche ist. Alternativ können der Kontaktclip und der Steuerclip unterschiedliche Steckrichtungen aufweisen, wobei besonders bevorzugt die Steckrichtungen um 90°, 180° oder 270° um das Kanülenrohr zueinander gedreht ausgerichtet sind.

Vorteilhaft ist es, wenn der Ansatz proximal einen Anschluss aufweist, an dem die zuzuspritzende Flüssigkeit koaxial zu dem Kanülenrohr angesetzt werden kann. Insbesondere ist es bevorzugt, dass der Anschluss mit einem in dem Ansatz eingesetzten Spritzschlauch ausgebildet ist. Der Zuspritzschlauch ist bevorzugt fest an dem Ansatz angeordnet und ist somit dort verlustsicher gehalten. Durch den Anschluss kann die Flüssigkeit, insbesondere ein Lokalanästhetikum in das Kanülenrohr eingeleitet werden, um diese Flüssigkeit über die Austrittsöffnung an der Kanülenspitze zu applizieren. Der Anschluss kann beispielsweise als Konnektor, insbesondere als Luer-Lock-Konnektor oder als NRfit-Konnektor ausgebildet sein. Dieser Konnektor kann unmittelbar an dem Ansatz ausgebildet sein oder alternativ an dem Zuspritzschlauch, der koaxial und abgedichtet an bzw. in dem Ansatz angesetzt ist.

Nach Maßgabe einer weiteren vorteilhaften Ausgestaltung der vorliegenden Erfindung ist das wenigstens eine Bedienelement in den Befestigungskörper eingelassen, wodurch das wenigstens eine Bedienelement vollumfänglich in dem Befestigungskörper hermetisch vor äußeren Einflüssen, insbesondere Flüssigkeiten oder Verunreinigungen, geschützt ist. Insbesondere ist dadurch eine Wiederverwendung des Steuerclips nach einer entsprechenden Reinigung möglich.

Weiterhin ist es besonders vorteilhaft, wenn das Bedienelement in der zweiten Aufbaustufe von der von dem Ansatz abgewandten Seite betätigbar ist, woraus sich eine besonders ergonomische Handhabbarkeit der erfindungsgemäßen Kanüle ergibt.

Auch hat es sich als vorteilhaft erwiesen, wenn der Befestigungskörper wenigstens einen verformbaren Bereich, insbesondere wenigstens einen elastisch verformbaren Bereich aufweist, und dass durch den wenigstens einen verformbaren Bereich das wenigstens eine in den Befestigungskörper eingelassene Bedienelement betätigbar ist. Die Betätigungsbewegung des medizinischen Personals wird durch den verformbaren Bereich auf das wenigstens eine Bedienelement übertragen, wodurch dieses entweder ein entsprechendes elektrisches Schaltsignal generiert oder mechanisch das Zuspritzen oder Aspirieren veranlasst wird.

Darüber hinaus ist nach Maßgabe einer vorteilhaften Ausgestaltung der vorliegenden Erfindung der Befestigungskörper aus wenigstens einem zweiten elektrisch isolierenden Kunststoff hergestellt, welcher eine gute Verformbarkeit aufweist. Vorzugsweise ist zumindest der wenigstens eine verformbare Bereich auf der von dem Ansatz abgewandten Seite mit dem zweiten elektrisch isolierenden Kunststoff hergestellt, wodurch das elektrische Bedienelement besonders leichtgängig betätigbar ist. Insbesondere haben sich als Werkstoff für den zweiten elektrisch isolierenden Kunststoff silikonhaltige Kunststoffe bewährt.

Vorteilhaft ist es, wenn das Bedienelement eine haptisch und/oder optisch und/oder akustisch wahrnehmbare Rückmeldung erzeugen kann. Insbesondere ist es vorteilhaft, wenn das Bedienelement zur Erzeugung einer haptischen und akustischen wahrnehmbaren Rückmeldung eine Schnappscheibe aufweist und/oder der Steuerclip eine oder mehrere visuell wahrnehmbare Signale, beispielsweise durch Leuchtmittel, insbesondere LEDs, erzeugt.

Darüber hinaus ist es vorteilhaft, wenn das wenigstens eine Bedienelement ein elektrisch kapazitiver Schalter und/oder ein elektrisch induktiver Schalter ist. Derartige Schalter sind kostengünstig und in der Handhabung sicher.

Gemäß einer weiteren vorteilhaften Ausgestaltung der vorliegenden Erfindung ist, bzw. sind der Ansatz und/oder das Befestigungsteil des Kontaktclips und/oder der Befestigungskörper des Steuerclips aus einem elektrisch isolierenden Kunststoff hergestellt. Insbesondere ist es dabei vorteilhaft, wenn der Ansatz, dass Befestigungsteil und der Befestigungskörper in einem wenigstens einen einkomponentigen Spritzgussverfahren hergestellt werden.

Nachfolgend wird unter Bezugnahme auf die begleitenden Zeichnungen ein beispielhaftes und erfindungsgemäßes Ausführungsbeispiel im Detail erläutert. Es zeigen:
- Figur 1: eine perspektivische Explosionsdarstellung der erfindungsgemäßen Kanüle, aufweisend ein Kanülenrohr mit einem am proximalen Ende des Kanülenrohrs angebrachten Ansatz, einen Kontaktclip und einen Steuerclip,
- Figur 2: eine perspektivische Darstellung der Kanüle gemäß Figur 1, wobei der Kontaktclip auf den Ansatz aufgesteckt ist,
- Figur 3: eine perspektivische Darstellung der Kanüle gemäß Figur 1 oder 2, wobei der Steuerclip zur Bildung einer ersten Aufbaustufe auf den Ansatz aufgesteckt ist,
- Figur 4: eine Schnittdarstellung entlang einer Längsachse durch die Kanüle gemäß Figur 3,
- Figur 5: eine Schnittdarstellung des Kanülenrohrs mit dem am proximalen Ende angebrachten Ansatz,
- Figur 6: eine Schnittdarstellung des Kontaktclips, und
- Figur 7: eine Schnittdarstellung des Steuerclips.

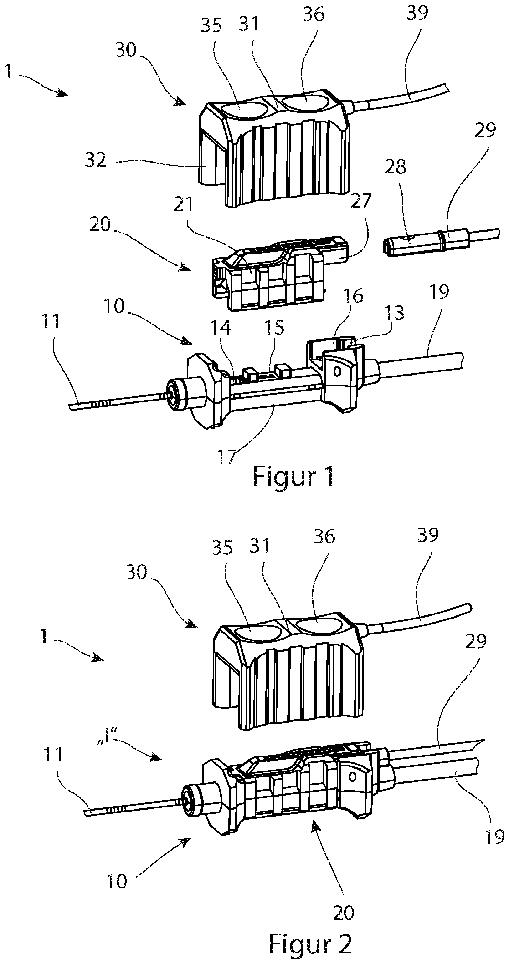

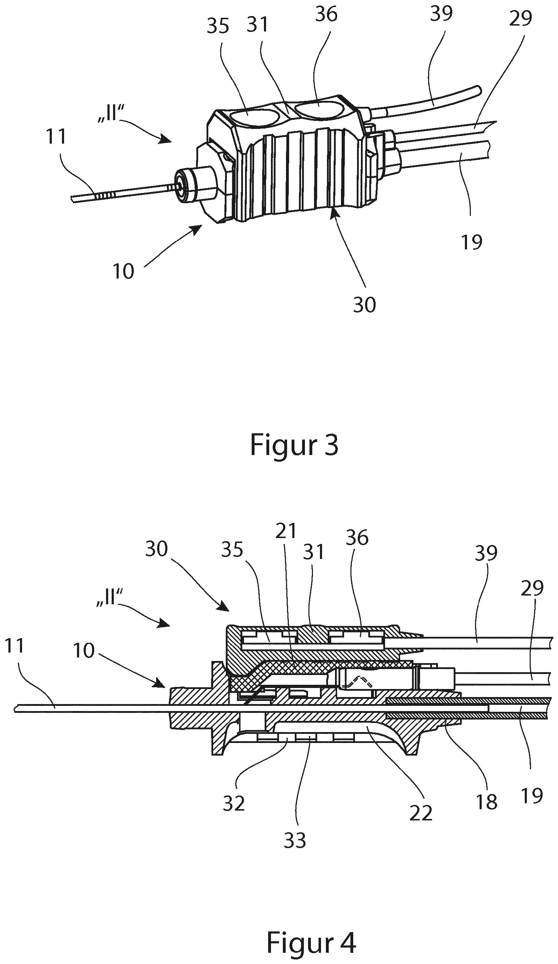

In den Figuren 1 bis 7 ist ein Ausführungsbeispiel einer erfindungsgemäßen Kanüle 1 dargestellt.

Die Kanüle 1 umfasst einen Kanülenkörper mit einem Kanülenrohr 11, das sich koaxial entlang einer Längsachse erstreckt. Das Kanülenrohr 11 kann aus einem elektrisch isolierenden Werkstoff hergestellt sein und kann darüber hinaus eine elektrisch leitfähige Ader aufweisen. Alternativ kann das Kanülenrohr 11 aus einem metallischen Werkstoff hergestellt sein und an seinem Außenumfang mit einem elektrisch isolierenden Kunststoff beschichtet sein.

Das Kanülenrohr 11 weist ein distales Ende und ein proximales Ende auf, wobei an dem distalen Ende eine (nicht dargestellte) Kanülenspitze ausgebildet ist, die eingerichtet ist, zu Behandlungszwecken in den Körper eines Patienten eingestochen zu werden. Am proximalen Ende des Kanülenrohrs 11 ist ein Ansatz 10 angeordnet, der bevorzugt aus einem elektrisch isolierenden Kunststoff auf das Kanülenrohr 11 aufgespritzt ist. Der Ansatz 10 hat im Wesentlichen die übliche Form eines rechteckigen Quaders, dessen proximales Ende und dessen distales Ende flanschförmig verbreitert sind, um ein sicheres Greifen und Führen der Kanüle durch ein medizinisches Fachpersonal zu ermöglichen. Zwischen dem Flansch an dem distalen Ende und dem Flansch am proximalen Ende ist eine Einsetzausnehmung 15 ausgebildet.

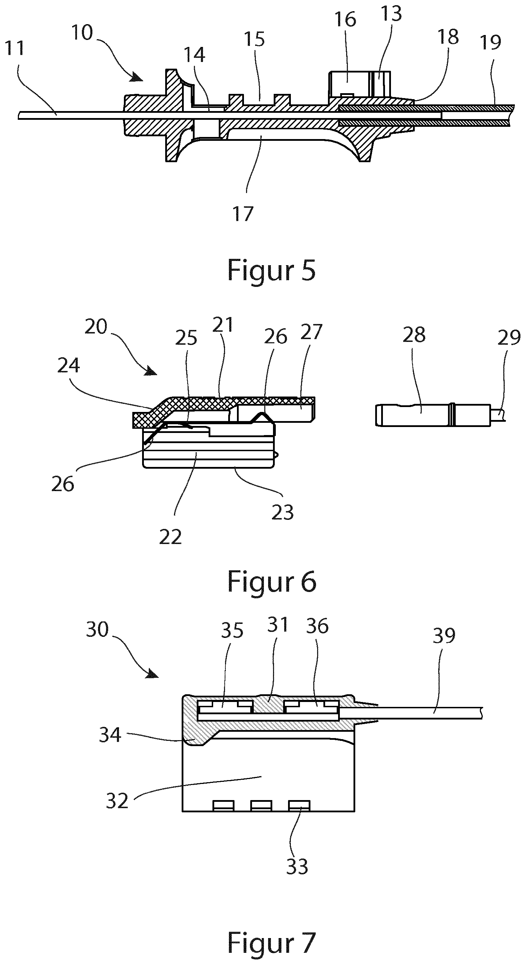

An dem proximalen Ende des Ansatzes 10 ist ein Anschluss 18 angeordnet. Der Anschluss 18 verbindet das Kanülenrohr 11 mit dem Zuspritzschlauch 19 - wie im Detail der Figur 5 zu entnehmen ist. Hierzu ist der Zuspritzschlauch 19 auf das proximale Ende, welches aus dem Ansatz 10 herausragt, hinübergeschoben und fest, sowie abgedichtet an dem Ansatz 10 angebracht bzw. befestigt.

Der Zuspritzschlauch 19 dient dem Zuführen und/oder Abführen einer Flüssigkeit, insbesondere eines Lokalanästhetikums, welches durch das Kanülenrohr 11 zu der Kanülenspitze an dem distalen Ende geleitet werden kann und am distalen Ende aus der Kanüle 1 austreten kann. Der Zuspritzschlauch kann an seinem (nicht dargestellten) freien Ende einen Anschluss aufweisen, z.B. einen Luer-Lock-Anschluss oder NRFit-Anschluss, an welchen eine Spritze oder dergleichen zum Zuführen der Flüssigkeit angeschlossen werden kann. Alternativ kann der Anschluss auch unmittelbar an dem Ansatz 10 bzw. am Anschluss 18 ausgebildet sein, wenn kein Zuspritzschlauch 19 erwünscht ist.

Wie den Figuren 1 und 5 zu entnehmen ist, weist der Ansatz 10 im Bereich der Einsetzausnehmung 15 zwischen dem distalen Flansch und dem proximalen Flansch eine Aussparung 14 auf, die auf der in der Zeichnung oberen Seitenfläche des Ansatzes 10 offen ist und in der Tiefe zumindest bis zu dem Kanülenrohr 11 reicht. In dem dargestellten Ausführungsbeispiel ist die Aussparung 14 diametral durchgehend durch den Ansatz 10 offen.

Das Kanülenrohr 11 kann im Bereich innerhalb der Aussparung 14 blank ausgebildet sein, wodurch das metallische Kanülenrohr 11 in diesem Abschnitt keine isolierende Umfangsbeschichtung aufweist.

Darüber hinaus sind in der Einsetzausnehmung 15 zwei von dem Kanülenrohr 11 abstehende Nocken ausgebildet, die einerseits beabstandet zu dem distalen Flansch und der Aussparung 14 und andererseits beabstandet zu dem proximalen Flansch ausgebildet sind.

An dem proximalen Ende des Ansatzes 10 ist in den proximalen Flansch eine Kerbe 16 eingearbeitet, welche sich von der Einsetzausnehmung 15 bis zum proximalen Ende durchgehend durch den proximalen Flansch und parallel zu dem Kanülenrohr 11 erstreckt.

Darüber hinaus umfasst die Kanüle 1 einen Kontaktclip 20 und einen Steuerclip 30, welcher beispielsweise in der Figur 1 in einer perspektivischen Ansicht dargestellt ist und in Figur 6 in einer Schnittdarstellung.

Der Kontaktclip 20 umfasst ein im Wesentlichen in Form eines rechteckigen Quaders ausgebildetes Befestigungsteil 21, in welches eine Ausnehmung eingeformt oder eingearbeitet ist, welche sich über die gesamte Längsausstreckung von einem proximalen Ende bis zu einem distalen Ende erstreckt und zwei diametral gegenüberliegende Rastarme 22 ausbildet. Dementsprechend ist der Kontaktclip 20 im Querschnitt U-förmig. Zwischen den beiden paarweise angeordneten Rastarmen 22 ist in der Ausnehmung des Befestigungsteils 21 ein elektrischer Kontakt 25 angeordnet. Der elektrische Kontakt 25 ragt in Richtung des distalen Endes des Kontaktclips 20 in die Ausnehmung zwischen den beiden Rastarmen 22, wobei das freie distale Ende des elektrischen Kontakts, welches in die Ausnehmung ragt, als Federzunge 26 ausgebildet ist und federnd parallel zu den beiden Rastarmen 22 ausgelenkt werden kann.

Weiterhin ist insbesondere Figur 5 zu entnehmen, dass das dem proximalen Ende zugewandte Ende des elektrischen Kontakts 25 ebenfalls als eine Federzunge 26 ausgebildet sein kann und in einen Bereich freistehend ragt, der als ein Steckverbinder 27 ausgebildet ist. In den Steckverbinder 27 kann das freie Ende eines Gegensteckverbinders 28 eingesteckt werden um eine elektrische Verbindung zwischen einem Stimulationskabel 29 und dem elektrischen Kontakt 25 herzustellen. Der Gegensteckverbinder 28 wird in dem Steckverbinder 27 gehalten, wobei die Federzunge 26 gegen einen elektrischen Gegenkontakt des Gegensteckverbinders 28 drückt und zum verlustsicheren Halten des Gegensteckverbinders 28 in dem Steckverbinder 27 beiträgt. Die Form des Steckverbinders 27 korrespondiert mit der Form des Gegensteckverbinders, wobei bevorzugt die dem Ansatz 10 zugewandte Seite des Steckverbinders 27 offen ist und durch den elektrischen Kontakt 25 bereichsweise verschlossen ist. Alternativ kann das Stimulationskabel 29 auch unmittelbar an dem elektrischen Kontakt 25 befestigt werden.

An den jeweiligen freien Ende der Rastarme 22 des Befestigungsteils 21 sind zueinander gerichtete Rastnasen 23 auf den Rastarmen 22 ausgebildet, die in die Ausnehmung ragen.

Um die Handhabbarkeit der Kanüle 1 zu verbessern, kann auf den Ansatz 10 ein Steuerclip 30 aufgesteckt bzw. aufgeclipst und so eine erste Aufbaustufe I gebildet werden. In dem dargestellten Ausführungsbeispiel ist jedoch vorgesehen, dass der Steuerclip 30 zur Bildung der ersten Aufbaustufe I den Kontaktclip 20 und den Ansatz 10 umgreift, wobei der Kontaktclip 20 zur Realisierung der ersten Aufbaustufe I nicht zwingend notwendig ist. In diesem Fall ist der Steuerclip 30 unmittelbar an dem Ansatz 10 befestigt.

Der Steuerclip 30 umfasst einen Befestigungskörper 31 und mindestens ein Bedienelement 35, 36. Im dargestellten Ausführungsbeispiel umfasst der Steuerclip 30 zwei elektrische Bedienelemente 35, 36, jedoch können die Bedienelemente ebenfalls mechanisch, beispielsweise als Ventile zum Steuern von Aspiration und Applikation ausgebildet sein.

Das erste Bedienelement 35 und das zweite Bedienelement 36 können voneinander unabhängig durch das medizinische Personal betätigt werden, wobei das erste Bedienelement 35 zum Aspirieren verwendet werden kann und das zweite Bedienelement 36 zur Regelung bzw. der Steuerung der zuzuspritzenden Flüssigkeit eingesetzt werden kann. Darüber hinaus kann bzw. können das erste Bedienelement 35 und/oder das zweite Bedienelement 36 zum Steuern der elektrischen Stimulationsimpulse verwendet werden.

Darüber hinaus kann der Steuerclip 30 eine Steuerleitung 39 umfassen, die entweder durch eine Steckverbindung löslich an dem Steuerclip 30 befestigbar ist oder fest mit dem Steuerclip 30 verbunden ist und eine elektrische Verbindung mit einer nicht dargestellten Einheit herstellt, welche beispielsweise die Aspiration oder das Zuspritzen bewirkt oder die elektrischen Stimulationsimpulse erzeugt, die durch das Stimulationskabel 29 mittels des elektrischen Kontakts 25 zu dem Kanülenrohr 11 geleitet werden.

Der Steuerclip 30 weist näherungsweise die übliche Form eines rechteckigen Quaders auf, in welchen eine sich von einem proximalen Ende zu einem distalen Ende erstreckende Aussparung eingearbeitet oder eingeformt ist, wodurch der Steuerclip 30 im Querschnitt U-förmig ausgebildet ist. Durch die Aussparung sind zwei diametral zueinander ausgerichtete Rastarme 32 ausgebildet, an deren freien Enden mehrere Rastnasen 33 angeformt oder angearbeitet sind, welche aufeinander gerichtet in die Aussparung ragen.

In den Befestigungskörper 31 sind die Bedienelemente 35, 36 eingelassen, was bedeutet, dass die Bedienelemente 35, 36 hermetisch von dem Befestigungskörper 31 umgeben sind, wodurch sichergestellt ist, dass keine Flüssigkeiten oder Verunreinigungen in Kontakt mit den Bedienelementen 35, 36 gelangen können.

Die Bedienelemente 35, 36 sind in den Figuren 1, 4, 6 auf der von dem Kanülenrohr 11 abgewandten Seite des Befestigungskörpers 31 angeordnet, und können somit während des Eingriffes mit der erfindungsgemäßen Kanüle 1 durch das medizinische Fachpersonal mit einer ersten Hand in ein und dergleichen Handhaltung betätigt werden, währenddessen dasselbe medizinische Fachpersonal mit der zweiten Hand den Ultraschallkopf hält und die Position der Kanüle bei der Behandlung, beispielsweise der Anästhesie beobachtet und überprüft. Durch die einfache Handhabbarkeit der Kanüle 1 kann sich das medizinische Fachpersonal mit voller Aufmerksamkeit dieser Aufgabe widmen.

Um eine einfache und kraftlose Betätigung der Bedienelemente 35, 36 zu ermöglichen, ist der Befestigungskörper 31 in einem Bereich auf der von dem Kanülenrohr 11 abgewandten Seite dünnwandig ausgebildet, wodurch eine mechanische Betätigung durch das medizinische Personal durch eine Verformung des Befestigungskörpers 31 auf die Bedienelemente 35, 36 übertragen wird. Insbesondere ist bevorzugt, wenn der verformbare Bereich aus einem weicheren Kunststoff, insbesondere Silikon hergestellt ist, wodurch der mechanische Widerstand beim Betätigen reduziert ist und die Handhabung verbessert ist. Besonders bevorzugt ist, wenn der Befestigungskörper 31 in einem Zweikomponenten-Spritzgussverfahren hergestellt wird.

Damit das medizinische Fachpersonal bei der Verwendung der erfindungsgemäßen Kanüle 1 die volle Aufmerksamkeit auf die Positionierung des Kanülenrohrs 11 an dem Einsatzort, beispielsweise einem Nerv des Patienten widmen kann, sieht eine vorteilhafte Weiterbildung der Erfindung vor, dass die Bedienelemente 35, 36 bei der Betätigung eine haptische, akustische oder visuelle Rückmeldung erzeugen, wobei vorteilhafter Weise die haptische und akustische Rückmeldung durch eine Schnappscheibe in dem jeweiligen Bedienelement 35, 36 erzeugt werden kann.

Soll die Kanüle 1 für die Elektrostimulation verwendet werden, so kann der Kontaktclip 20 auf den Ansatz 10 angebracht bzw. aufgeclipst werden und der Kontaktclip 20 und der Kanülenkörper mit dem Ansatz 10 und dem Kanülenrohr 11 bilden zusammen die zweite Aufbaustufe II. Der Kontaktclip 20 wird in einer Steckrichtung senkrecht zu dem Kanülenrohr 11 auf den Ansatz aufgesteckt.

Die zweite Aufbaustufe II ist perspektivisch in Figur 2 dargestellt und es ist ersichtlich, dass in der zweiten Aufbaustufe I der Kontaktclip 20 seitlich in der Steckrichtung in die Einsetzausnehmung 15 eingesetzt ist und die beiden Rastarme 22 den Ansatz 10 zwischen dem distalen Flansch und dem proximalen Flansch umgreifen, wobei die Rastnasen 23 zur Bildung einer formschlüssigen Schnapp- bzw. Clipsverbindung den Ansatz 10 bzw. eine Kante 17 auf der von dem Kontaktclip 20 abgewandten Seite des Ansatzes 10 bereichsweise hintergreifen.

In der zweiten Aufbaustufe II ragt die Federzunge 26 des elektrischen Kontakts 25 in die Aussparung 14 des Ansatzes 10 zwischen dem distalen Flansch und dem proximalen Flansch und drückt mit seiner an dem freien Ende ausgebildeten Federzunge 26 gegen das Kanülenrohr 11, wodurch eine elektrische Verbindung zwischen dem elektrischen Kontakt 25 und dem Kanülenrohr 11 bewerkstelligt ist.

Darüber hinaus drücken die beiden in der Einsetzausnehmung 15 angeordneten Nocken gegen den elektrischen Kontakt 25. Dadurch kann eine zusätzliche Spannkraft auf die zweite Federzunge 26 an dem proximalen Ende des Kontaktclips 20 erreicht werden, wodurch der Gegensteckverbinder 28 in dem Steckverbinder 27 verspannt gehalten wird und ein Lösen der elektrischen Verbindung zwischen dem Stimulationskabel 29 und dem elektrischen Kontakt 25 verhindert ist. Das proximale Ende des Kontaktclips 20 taucht vollständig in die Kerbe 16 am Ansatz 10 ein. Darüber hinaus sind in der Kerbe 16 zwei Haltestege 13 ausgebildet, die von dem Ansatz 10 in die Kerbe 16 ragen und einen Steg an dem Gegensteckverbinder 28 bereichsweise umgreifen und den Gegensteckverbinder in der Kerbe 16 festlegen.

Zum Überführen der Kanüle 1 in die erste Aufbaustufe I wird der Steuerclip 30 in die Steckrichtungen im dargestellten Ausführungsbeispiel über die zweite Aufbaustufe I bzw. den Ansatz 10 und den Konaktclip 20 aufgesetzt, wobei die beiden Rastarme 32 den Kontaktclip 20 diametral umgreifen und die an dem freien Ende der Rastarme 32 angeordneten Rastnasen 33 einen Formschluss mit dem Kontaktclip 20 bilden, in dem diese teilweise den Kontaktclip 20 bzw. das freie Ende der Rastarme 22 des Kontaktclips 20 umgreifen. Dabei wird die Position des Steuerclips 30 zwischen dem distalen Flansch und dem proximalen Flansch des Ansatzes 10 durch das Zusammenwirken einer Vertiefung 24 an dem distalen Ende des Kontaktclips 20 und einem Vorsprung 34 an dem distalen Ende des Steuerclips 30 sowie dem distalen Flansch an dem Ansatz 10 festgelegt, die im zusammengesetzten Zustand in der ersten Aufbaustufe I ineinandergreifen.

### Bezugszeichenliste

- 1: Kanüle
- 10: Ansatz
- 11: Kanülenrohr
- 13: Haltesteg
- 14: Aussparung
- 15: Einsetzausnehmung
- 16: Kerbe
- 17: Kante
- 18: Anschluss
- 19: Zuspritzschlauch
- 20: Kontaktclip
- 21: Befestigungsteil
- 22: Rastarm
- 23: Rastnase
- 24: Vertiefung
- 25: Kontakt
- 26: Federzunge
- 27: Steckverbinder
- 28: Gegensteckverbinder
- 29: Stimulationskabel
- 30: Steuerclip
- 31: Befestigungskörper
- 32: Rastarm
- 33: Rastnase
- 34: Vorsprung
- 35: Bedienelement
- 36: Bedienelement
- 39: Steuerleitung

## Patentansprüche

1. Kanüle (1), umfassend:
- einen Kanülenkörper mit einem Kanülenrohr (11) mit einer Kanülenspitze am distalen Ende und einem am proximalen Ende des Kanülenrohrs (11) angebrachten Ansatz (10),
- einen Steuerclip (30), aufweisend einen Befestigungskörper (31) mit wenigstens einem Bedienelement (35, 36),
- wobei der Steuerclip (30) seitlich an dem Ansatz (10) zur Bildung einer ersten Aufbaustufe (I) anbringbar ist, wobei
der Ansatz (10) proximal einen Anschluss (18) aufweist, an dem die zuzuspritzende Flüssigkeit koaxial zu dem Kanülenrohr (11) angesetzt werden kann,
**dadurch gekennzeichnet, dass** ein Kontaktclip (20) vorgesehen ist, und dass der Kontaktclip (20) wenigstens ein Befestigungsteil (21) mit wenigstens einem elektrischen Kontakt (25) aufweist, und dass der Kontaktclip (20) zur Bildung einer zweiten Aufbaustufe (II) seitlich an dem Ansatz (10) anbringbar ist und der elektrische Kontakt (25) in der zweiten Ausbaustufe (II) eine elektrische Verbindung zwischen einem Stimulationskabel (29) und einer elektrisch leifähigen Ader des Kanülenrohrs (11) im Bereich des Ansatzes (10) herstellt.

2. Kanüle (1) nach Anspruch 1,
**dadurch gekennzeichnet, dass** das Befestigungsteil (21) des Kontaktclips (20) wenigstens ein Paar von federnden Rastarmen (22) aufweist, die zur Herstellung einer Rastverbindung in der zweiten Aufbaustufe (II) den Ansatz (10) umgreifen.

3. Kanüle (1) nach Anspruch 1 oder 2,
**dadurch gekennzeichnet, dass** in der zweiten Aufbaustufe (II) der elektrische Kontakt in eine Aussparung (14) des Ansatzes (10) eingreift und das Kanülenrohr (11) elektrisch kontaktiert.

4. Kanüle (1) nach einem der Ansprüche 1 bis 3,
**dadurch gekennzeichnet, dass**
der elektrische Kontakt (25) einen Schneidklemmkontakt aufweist, der auf das Kanülenrohr (11) gedrückt wird.

5. Kanüle (1) nach einem der Ansprüche 1 bis 4,
**dadurch gekennzeichnet, dass**
der elektrische Kontakt (25) eine Federzunge (26) aufweist, die sich in der Aussparung (14) des Ansatzes (10) an das Kanülenrohr (11) anlegt.

6. Kanüle (1) nach einem der Ansprüche 1 bis 5,
**dadurch gekennzeichnet, dass**
der Kontaktclip (20) einen Steckverbinder (27) aufweist, der eine elektrisch lösliche Verbindung mit dem Stimulationskabel (29) herstellen kann.

7. Kanüle (1) nach einem der Ansprüche 1 bis 6,
**dadurch gekennzeichnet, dass**
der elektrische Kontakt (25) eine Federzunge (26) zur Bildung eines Klemmkontakts mit dem elektrischen Steckverbinder aufweist.

8. Kanüle (1) nach einem der vorgenannten Ansprüche,
**dadurch gekennzeichnet, dass**
das wenigstens eine Bedienelement (35, 36) ein elektrisches Bedienelement ist und mit einer Steuerleitung (39) verbunden ist.

9. Kanüle (1) nach einem der vorgenannten Ansprüche,
**dadurch gekennzeichnet, dass**
der Steuerclip (30) wenigstens zwei Bedienelemente (35, 36) umfasst.

10. Kanüle (1) nach einem der vorgenannten Ansprüche,
**dadurch gekennzeichnet, dass**
der Befestigungskörper (31) des Steuerclips (30) wenigstens zwei Rastarme (32) umfasst, die den Ansatz (10) und/oder das Befestigungsteil (21) des Kontaktclips (20) umgreifen.

11. Kanüle (1) nach einem der vorgenannten Ansprüche,
**dadurch gekennzeichnet, dass**
der Anschluss (18) zum Einleiten der Flüssigkeit koaxial an dem Ansatz zu dem Kanülenrohr angesetzt ist.

12. Kanüle (1) nach einem der vorgenannten Ansprüche,
**dadurch gekennzeichnet, dass**
der Anschluss (18) mit einem in den Ansatz (10) eingesetzten Zuspritzschlauch (19) ausgebildet ist.

13. Kanüle (1) nach einem der vorgenannten Ansprüche,
**dadurch gekennzeichnet, dass**
der Befestigungskörper (31) das wenigstens eine Bedienelement (35, 36) umgibt, und dass das wenigstens eine Bedienelement (35, 36) von der von dem Ansatz (11) abgewandten Seite betätigbar ist.

14. Kanüle (1) nach einem der vorgenannten Ansprüche,
**dadurch gekennzeichnet, dass**
der Befestigungskörper auf der von dem Ansatz (10) abgewandten Seite zur Betätigung des Bedienelements (35, 36) verformbar ist und/oder dass der Befestigungskörper (31) auf der von dem Ansatz (10) abgewandten Seite zur Betätigung des Bedienelements (35, 36) aus einem zweiten elektrisch isolierenden Kunststoff hergestellt ist, welcher leicht verformbar ist, vorzugsweise Silikon.

15. Kanüle (1) nach einem der vorgenannten Ansprüche,
**dadurch gekennzeichnet, dass** beim Betätigen des Bedienelements (35, 36) eine haptische Rückmeldung, insbesondere durch eine Schnappscheibe erzeugt ist.

16. Kanüle (1) nach einem der vorgenannten Ansprüche,
**dadurch gekennzeichnet, dass**
das wenigstens eine Bedienelement (35, 36) einen elektrischen kapazitiven Schalter und/oder einen elektrischen induktiven Schalter umfasst.

17. Kanüle (1) nach einem der vorgenannten Ansprüche,
**dadurch gekennzeichnet, dass**
der Ansatz (10) und/oder das Befestigungsteil (21) des Kontaktclips (20) und/oder der Befestigungskörper (31) des Steuerclips (30) aus einem elektrisch isolierenden Kunststoff hergestellt sind.

## Claims

1. Cannula (1), comprising:
- a cannula body having a cannula tube (11), with a cannula tip at its distal end and with an attachment (10), attached at the proximal end of the cannula tube (11),
- a control clip (30), comprising a fixing body (31) which has at least one control element (35, 36),
- wherein the control clip (30) is laterally attachable to the attachment (10) in order to form a first assembly step (I), wherein
the attachment (10) comprises proximally a connection (18), into which the liquid to be injected can be, coaxially to the cannula tube (11), conducted,
**characterized in that**
a contact clip (20) is provided, and **in that** the contact clip (20) comprises at least one fixing part (21) having at least one electrical contact (25), and **in that** the contact clip (20) is laterally attachable to the attachment (10), in order to form a second assembly step (II) and **in that** the electrical contact (25) establishes, in the second assembly step (II), an electrical connection between a stimulation cable (29) and an electrically conductive core of the cannula tube (11) in the region of the attachment (10).

2. Cannula (1) in accordance with claim 1,
**characterized in that**
the fixing part (21) of the contact clip (20) comprises at least one pair of resilient locking arms (22), which engage around the attachment (10) in order to form locking connection in the second assembly step (II).

3. Cannula (1) in accordance with claim 1 or 2,
**characterized in that**
in the second assembly step (II), the electrical contact engages in a recess (14) of the attachment (10) and electrically contacts the cannula tube (11).

4. Cannula (1) in accordance with any of claims 1 to 3,
**characterized in that**
the electrical contact (25) comprises an insulation displacement contact, which is pressed onto the cannula tube (11).

5. Cannula (1) in accordance with any of claims 1 to 4,
**characterized in that**
the electrical contact (25) comprises a spring tongue (26) which rests against the cannula tube (11) in the recess (14) of the attachment (10).

6. Cannula (1) in accordance with any of claims 1 to 5,
**characterized in that**
the contact clip (20) comprises a plug connector (27) which can establish an electrically disconnectable connection to the stimulation cable (29).

7. Cannula (1) in accordance with any of claims 1 to 6,
**characterized in that**
the electrical contact (25) comprises a spring tongue (26) in order to form a clamping contact with the electrical plug connector.

8. Cannula (1) in accordance with any of the preceding claims,
**characterized in that**
the at least one control element (35, 36) is an electrical control element and is connected to a control wire (39).

9. Cannula (1) in accordance with any of the preceding claims,
**characterized in that**
the control clip (30) comprises at least two control elements (35, 36).

10. Cannula (1) in accordance with any of the preceding claims,
**characterized in that**
the fixing body (31) of the control clip (30) comprises at least two resilient locking arms (32) which engage around the attachment (10) and/or the fixing part (21) of the contact clip (20).

11. Cannula (1) in accordance with any of the preceding claims,
**characterized in that**
the connection (18) for conducting the liquid is disposed on the attachment coaxially to the cannula tube.

12. Cannula (1) in accordance with any of the preceding claims,
**characterized in that**
the connection (18) is implemented having an injection tube (19) inserted in the attachment (10).

13. Cannula (1) in accordance with any of the preceding claims,
**characterized in that**
the fixing body (31) encloses the at least one control element (35, 36), and **in that** the at least one control element (35, 36) can be operated from the side facing away from the attachment (11).

14. Cannula (1) in accordance with any of the preceding claims,
**characterized in that**
the fixing body is deformable on the side facing away from the attachment (10) for operating the control element (35, 36), and/or **in that** the fixing body (31) is, on the side facing away from the attachment (10), manufactured from a second electrically conductive plastic which is more easily deformable, preferably silicone, for operating the control elements (35, 36).

15. Cannula (1) in accordance with any of the preceding claims,
**characterized in that**
when operating the control elements (35, 36), a haptic response is provoked, in particular by means of a snap disc.

16. Cannula (1) in accordance with any of the preceding claims,
**characterized in that**
the at least one control element (35, 36) comprises an electrical capacitive switch and/or an electrical inductive switch.

17. Cannula (1) in accordance with any of the preceding claims,
**characterized in that**
the attachment (10) and/or the fixing part (22) of the contact clip (20) and/or of the fixing body (31) of the control clip (30) is(are) manufactured from an electrically insulating plastic.

## Revendications

1. Canule (1) comprenant :
- un corps de canule avec un tube de canule (11) ayant une pointe de canule à son extrémité distale et un embout (10) à l'extrémité proximale du tube de canule (11),
- un clip de contrôle (30) comprenant un corps de fixation (31) avec au moins un élément de commande (35, 36),
- le clip de contrôle (30) pouvant se fixer latéralement à l'embout (10) pour former un premier niveau d'assemblage (1),
dans lequel
l'embout (10) comporte côté proximal, un raccord (18) recevant le liquide à injecter co-axialement au tube de canule (11),
canule **caractérisée par**
un clip de contact (20), et
le clip de contact (20) comporte au moins une pièce de fixation (21) avec au moins un contact électrique (25), et
- le clip de contact (20) se met latéralement sur l'embout (10) pour former un second niveau d'assemblage (II) et le contact électrique (25), dans le second niveau d'assemblage (II), forme une liaison électrique entre un câble de stimulation (29) et le fil conducteur du tube de canule (11) dans la région de l'embout (10).

2. Canule (1) selon la revendication 1,
**caractérisée en ce que**
la pièce de fixation (21) du clip de contact (20) comporte au moins une paire de bras élastiques d'accrochage (22), qui entourent l'embout (10) pour réaliser une liaison par accrochage pour le second niveau d'assemblage (II).

3. Canule (1) selon la revendication 1 ou 2,
**caractérisée en ce que**
dans le second niveau d'assemblage (II), le contact électrique pénètre dans un évidement (14) de l'embout (10) et est en contact électrique avec le tube de canule (11).

4. Canule (1) selon l'une des revendications 1 à 3,
**caractérisée en ce que**
le contact électrique (25) comporte un contact à couteau qui se presse sur le tube de canule (11).

5. Canule (1) selon l'une des revendications 1 à 4,
**caractérisée en ce que**
le contact électrique (25) comporte une languette élastique (26) qui s'applique contre le tube de canule (11) dans l'évidement (14) de l'embout (10).

6. Canule (1) selon l'une des revendications 1 à 5,
**caractérisée en ce que**
le clip de contact (20) comporte un connecteur d'enfichage (27) réalisant une liaison électrique amovible avec le câble de stimulation (29).

7. Canule (1) selon l'une des revendications 1 à 6,
**caractérisée en ce que**
le contact électrique (25) comporte une languette élastique (26) pour former un contact par serrage avec le connecteur électrique.

8. Canule (1) selon l'une des revendications précédentes,
**caractérisée en ce que**
au moins un élément de commande (35, 36) est un élément de commande électrique relié à une ligne de commande (39).

9. Canule (1) selon l'une des revendications précédentes,
**caractérisée en ce que**
le clip de contrôle (30) comprend au moins deux éléments de commande (35, 36).

10. Canule (1) selon l'une des revendications précédentes,
**caractérisée en ce que**
le corps de fixation (31) du clip de commande (30) comporte au moins deux bras d'accrochage (32) qui entourent l'embout (10) et/ou la pièce de fixation (21) du clip de contact (20).

11. Canule (1) selon l'une des revendications précédentes,
**caractérisée en ce que**
le raccord (18) s'applique sur le tube de canule pour introduire le liquide coaxialement à l'embout.

12. Canule (1) selon l'une des revendications précédentes,
**caractérisée en ce que**
le raccord (18) est réalisé avec un tuyau d'injection (19) mis dans l'embout (10).

13. Canule (1) selon l'une des revendications précédentes,
**caractérisée en ce que**
le corps de fixation (31) entoure au moins un élément de commande (35, 36), et
cet élément de commande (35, 36) peut s'actionner à partir du côté opposé à celui de l'embout (11).

14. Canule (1) selon l'une des revendications précédentes,
**caractérisée en ce que**
le corps de fixation est déformable sur le côté opposé à l'embout (10) pour actionner l'élément de commande (35, 36) et/ou le corps de fixation (31) est réalisé en une seconde matière plastique électro-isolante, sur le côté opposé à l'embout (10) pour actionner l'élément de commande (35, 36), cette matière plastique facilement déformable, étant de préférence du silicone.

15. Canule (1) selon l'une des revendications précédentes,
**caractérisée en ce que**
l'actionnement de l'élément de commande (35, 36) génère un signal de retour haptique, notamment par une rondelle d'enclipsage.

16. Canule (1) selon l'une des revendications précédentes,
**caractérisée en ce que**
au moins un élément de commande (35, 36) comprend un commutateur électrique capacitif et/ou un commutateur électrique inductif.

17. Canule (1) selon l'une des revendications précédentes,
**caractérisée en ce que**
l'embout (10) et/ou la pièce de fixation (21) du clip de contact (20) et/ou du corps de fixation (31) du clip de contrôle (30) sont réalisés en une matière plastique électro-isolante.
